# EUROPÄISCHE PATENTSCHRIFT

(11) **EP 1 067 195 B1**
(45) Veröffentlichungstag und Bekanntmachung des Hinweises auf die Patenterteilung: **20.09.2006**
(21) Anmeldenummer: 00113127.5
(22) Anmeldetag: 29.06.2000
(51) Int. Cl.: C12P 7/04, C12P 7/18, C12R 1/225, C12R 1/24

(54) **Verfahren zur Reduktion von Ketogruppen enthaltende Verbindungen**
Process for reducing keto-group containing compounds
Procédé de réduction des composés contenant un groupe cétonique

(30) Priorität: 09.07.1999 DE 19932040
(43) Veröffentlichungstag der Anmeldung: 10.01.2001
(73) Patentinhaber: Forschungszentrum Jülich GmbH, 52425 Jülich (DE)
(72) Erfinder: Hummel, Werner, 52445 Titz (DE); Liese, Andreas, 52428 Jülich (DE); Wandrey, Christian, 52428 Jülich (DE)
(74) Vertreter: Fitzner, Uwe

(56) Entgegenhaltungen:
- EP-A- 0 456 107
- EP-A- 0 796 914
- BESSE P ET AL: "Microbiological reductions of chromen-4-one derivatives" TETRAHEDRON: ASYMMETRY, ELSEVIER SCIENCE PUBLISHERS, AMSTERDAM, NL, Bd. 10, Nr. 24, 17. Dezember 1999 (1999-12-17), Seiten 4745-4754, XP004188778 ISSN: 0957-4166
- OCCHIATO E G ET AL: "Baker's Yeast Reduction of Prochiral gamma-Nitroketones. II. Straightforward Enantioselective Synthesis of 2,7-Dimethyl-1,6-dioxaspiro[4.4]nonanes" TETRAHEDRON: ASYMMETRY, ELSEVIER SCIENCE PUBLISHERS, AMSTERDAM, NL, Bd. 6, Nr. 12, 1. Dezember 1995 (1995-12-01), Seiten 2971-2976, XP004047947 ISSN: 0957-4166

## Beschreibung

Die vorliegende Erfindung betrifft ein Verfahren zur Reduktion von carbonsäure- oder estergruppenfreien Ketogruppen enthaltenden Verbindungen zu Hydroxyverbindungen.
Optisch aktive Hydroxyverbindungen sind wertvolle chirale Bausteine. So sind chirale Diole wichtige Grundstoffe für eine Vielzahl von Wirkstoffen in der Pharmazie und im Pflanzenschutz sowie für Katalysatoren. Beispielsweise enthält der Homogenkatalysator Me-DuPHOS enantiomerenreines (2*S*, 5*S*)-Hexandiol.
Chemische Synthesemethoden zur gezielten Darstellung chiraler Diole sind trotz zahlreicher veröffentlichter Versuche offensichtlich für die Herstellung der genannten Produkte nicht ausreichend geeignet. Zur Synthese von enantiomerenreinem Hexandiol haben beispielsweise Versuche ergeben, daß die direkte Reduktion nur ein Produkt mit geringer optischer Reinheit ergibt (Bach, J. et al. (1997) Tetrahedron Letters 38: 1091-1094; Fan, Q.-H. et al. (1997) Tetrahedron: Asymmetry 8:4041-45).
Andere Verfahren beinhalten mehrstufige Synthesen, z.B. wird in einem üblichen Verfahren zunächst ein beta-Ketoester mit Noyoris BINAP-Reagenzien (Burk, M. J. et al. (1991) Tetrahedron: Asymmetry 2:569-592) reduziert. Nach anschließender Verseifung wird eine KOLBE-Kupplung durchgeführt. Dieses elektrochemische Verfahren ist im Labor gut anwendbar, leidet aber unter dem Problem des Elektrodenfoulings bei der Maßstabsvergrößerung. Eine andere bekannte Synthese benötigt fünf Stufen ausgehend vom Mannitol (Saravanan, P. et al. (1997) J. Org. Chem. 62:2669-2670).

Zur Herstellung von chiralen Verbindungen werden daher zunehmend biotechnologische Verfahren angewendet, die entweder unter Verwendung ganzer Mikroorganismen-Zellen oder mittels isolierter Enzyme arbeiten.
Enzyme haben den Vorteil, daß Umsetzungen in aller Regel ohne Nebenprodukte und mit hoher Enantiomerenreinheit ablaufen. Geeignete Enzyme sind Oxidoreduktasen zur Reduktion der prochiralen Keton-Vorstufe (z.B. NAD(P)-abhängige Dehydrogenasen) oder auch Hydrolasen, die durch Racemattrennung zur Darstellung chiraler Diole eingesetzt werden können (Bianchi, D. et al. (1993) Indian J. Chem., Sect B, 32B:176-80; Bosetti, A. et al. (1992) J. Chem. Soc. Perkin Trans. l:2395-2398; Caron, G. und Kazlauskas, R. J. (1993) Tetrahedron:Asymmetry, 4:1995-2000; Hof, R. P. und Kellogg, R. M. (1994) Tetrahedron:Asymmetry 5:565-568; Theil, F. et al. (1994) J. Org. Chem. 59:388-393).
Der Einsatz von Lipasen ist allerdings unter ökonomischen Gesichtspunkten in diesem speziellen Fall nicht attraktiv, da bei der kinetischen Racematspaltung mit einer Lipase lediglich eine maximale Ausbeute von 50% erzielt werden kann. Bei der Darstellung von diastereomerenreinen Diolen erniedrigt sich die maximale erreichbare Ausbeute dann sogar auf 25%. Trotz dieser niedrigen erreichbaren Ausbeute ist die Herstellung von 2,5-Hexandiol im kg-Maßstab durch Lipase-katalysierte Racematspaltung beschrieben (Nagai, H. et al. (1994) Synlett 4:289-90). Dieses Verfahren weist bei einer theoretischen maximalen Ausbeute von 25% aber tatsächlich nur eine Ausbeute von 17% auf. Es fallen demgemäß große Mengen an unerwünschten Nebenprodukten an.
Geeignete Oxidoreduktasen zur Synthese von Alkoholen sind in der EP 0456107A2 (gewonnen aus Lactobacillus kefir), der EP 0796914A2 und der EP 1002097A2 (aus Lactobacillus brevis) beschrieben. Sie erfordern allerdings die Zugänglichkeit eines geeignete Enzyms, den Zusatz eines löslichen Coenzyms (NADH, NADPH) und ein Coenzym-Regenerierungssystem. Ebenso wird in der WO 97/00968 der Einsatz von Reduktasen für die Reduktion von Ketogruppen beschrieben.
Ferner wird in der US-PS 5,342,767 ein Verfahren beschrieben, bei dem unter Einsatz von Alkohol-Dehydrogenase aus Lactobacillus kefir eine Reduktion durchgeführt wird.
In der DE 196 10 984.1 wird ebenfalls ein Verfahren beschrieben, bei dem Alkohol-Dehydrogenase zum Einsatz kommt. Neben den gereinigten Enzymen können auch ganze Zellen verwendet werden. Gegenstand dieser Erfindung ist jedoch nicht die Umsetzung von Verbindungen mit zwei oder mehr Ketogruppen.

Mikroorganismen stellen eine kostengünstige Alternative zu Enzymen dar. Allerdings ergeben diese Verfahren häufig niedrige Ausbeuten (F. Aragazzini et al., Appl. Microbiol. Biotechnol. (1986) 24, 175-177). Außerdem treten häufig ausbeutemindernde Nebenreaktionen auf und die Produkte besitzen nicht immer eine ausreichende Enantiomerenreinheit. Die Produktausbeute und -qualität hängen stark vom verwendeten Stamm und den Anzuchtbedingungen ab.
Seit langem ist z.B. die Reduktion von Ketonen bekannt. Solche Synthesen werden im Labormaßstab bereits seit mehr als 50 Jahren durchgeführt. Auch Diketone sind häufiger als Substrate verwendet worden, insbesondere α-Diketone verschiedenster Strukturen wurden mit diesem Verfahren zu chiralen Diolen oder Hydroxy-Ketonen reduziert, z.B. zu Benzilen, Benzoinen, α,β-Diketodithianen, α-Hydroxy-Ketonen und azyklischen α-Diolen. So kann man beispielsweise mit Bäckerhefe (*R*)-(-)-1,2-Propandiol durch Reduktion der Ketonvorstufe mit einem ee-Wert von 98% und einer Ausbeute von 95% erhalten (Kometani, T. et al. (1993) J. Ferment. Bioeng. 76:414-415), (-)1,3,3-Trimethyl-Cyclohexan-1,2-Diol mit einem ee-Wert von größer als 90% und einer Ausbeute von 36% (Negi, M. et al. (1993) Enzyme Microb. Technol. 15:483-488). Auch Diole, bei denen die Alkoholgruppen in einem größeren Abstand zueinander stehen (1,3-,1,4-Diole) sind durch Reduktion mit Bäckerhefe erhalten worden, z.B. enantiomerenreines (S,S)-2,5-Hexandiol nach 6 Tagen mit einer Ausbeute von 90% (Lieser, J. K. (1983) Synthetic Communications13:765-767) oder (S,S)-5-Nitro-2,8-Nonandiol mit einem ee-Wert von 100% und einer Ausbeute von 58% nach drei Tagen (Occhiato, E. G. et al. (1995) Tetrahedron:Asymmetry 6:2971-6).
Werden Diketone, wie Hexandion mit Bäckerhefe reduziert, erhält man offensichtlich immer (*S*,*S*)-Diole. Für (*R*,*R*)-Diole, beispielsweise (*R*,*R*)-Hexandiol sind bisher keine Mikroorganismen mit entsprechend reduzierender Aktivität bekannt.
Aus der europäischen Patentanmeldung 0569 998 A2 ist schließlich ein Verfahren bekannt, bei dem verschiedene Mikroorganismen zur Reduktion von Ethergruppen enthaltenden Diketoestern eingesetzt werden. Hierbei werden die Diketoester in 5-Position, in 3-Position oder in 3- und 5-Position reduziert. Zu den nach der Beschreibung dieser Erfindung einsetzbaren Mikroorganismen zählen zahlreiche Hefen und Bakterien, nicht jedoch Lactobacillus-Arten.

Eine Reduktion zu enantioselektiv und diastereoselektiv reduzierten Polyhydroxyverbindungen ist mit den bisherigen Verfahren in Gegenwart von Enzymen und den bisher bekannten Verfahren mit Hilfe verschiedener Mikroorganismen nicht möglich.

Aufgabe der vorliegenden Erfindung ist es demgemäß, ein Verfahren zur Reduktion von carbonsäure- und estergruppenfreien Verbindungen zur Verfügung zu stellen, das die geschilderten Nachteile nicht mehr aufweist und insbesondere eine umweltfreundliche und kostengünstige Herstellung von Polyhydroxyverbindungen erlaubt.

Diese Aufgabe wird erfindungsgemäß gelöst durch ein Verfahren zur Reduktion von wenigstens zwei Ketogruppen enthaltenden und carbonsäuregruppen- oder estergruppenfreien Verbindungen, bei dem in Gegenwart von Lactobacillus-Arten wenigstens eine Ketogruppe zu einer Hydroxylgruppe umgesetzt wird.
Bevorzugt werden erfindungsgemäß ethergruppenfreie Verbindungen zur Reduktion eingesetzt.
Vorzugsweise wird erfindungsgemäß angestrebt, daß eine Reduktion von wenigstens zwei Ketogruppen zu wenigstens zwei Hydroxylgruppen stattfindet. In einer besonders bevorzugten Ausführungsform wird eine Reduktion zu Diolen durchgeführt.
Das Verfahren nach der vorliegenden Erfindung beinhaltet insbesondere die katalytische Reduktion der prochiralen Dioxoverbindungen gemäß Formel I: 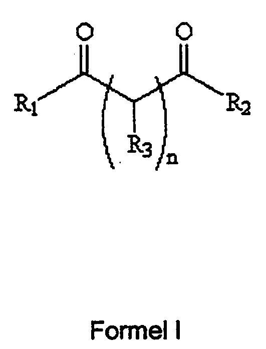 R₁, R₂ = eine Komponente aus der Gruppe Alkyl, Alkenyl, Cycloalkyl, Cycloalkenyl, Aryl, Aralkyl, Cycloalkylalkyl, wobei die Komponenten auch mit Heteroatomen, wie z.B. Si, N, P, O, S, F, Cl, Br, und I substituiert sein können.
R₃ = R₁, R₂, H, Halogene oder andere Heterokomponenten, z.B. Amine n = 0 - 10.

Unter Alkyl sind sowohl geradkettige als auch verzweigte gesättigte Kohlenstoff-Ketten zu verstehen. Beispielhaft können Methyl, Ethyl, n-Propyl, i-Propyl, t-Butyl, Pentyl, i-Pentyl, n-Hexyl, i-Hexyl genannt werden.
Mit Alkenyl sind geradkettige und verzweigte ungesättigte Kohlenwasserstoffe bezeichnet, für die beispielhaft Vinyl, 1-Propenyl, Allyl, Butenyl, i-Butenyl genannt werden können.
Von dem Begriff Cycloalkyl werden gesättigte, ringförmige Kohlenwasserstoffketten umfaßt, die aus drei, vier, fünf, sechs oder sieben Kohlenwasserstoffatomen bestehen.
Cycloalkenyl bezeichnet ungesättigte, ringförmige Kohlenwasserstoffe, mit fünf, sechs, sieben oder acht Kohlenstoffatomen. Unter Aryl sind aromatische Systeme, eingeschlossen Heteroaromaten und substituierte aromatische Systeme, wie Phenyl, zu verstehen.
Unter Aralkyl sind Arylreste zu verstehen, die über Alkyl Gruppen angebunden sind, z. B. ein Benzylrest.
Unter den Begriff Cycloalkylalkyl fallen Cycloalkylreste, die über Alkylgruppen gebunden sind.
Unter den Halogenen sind Fluor und Chlor bevorzugt.
Mit dem erfindungsgemäßen Verfahren können demgemäß insbesondere folgende Hydroxyverbindungen erzeugt werden:

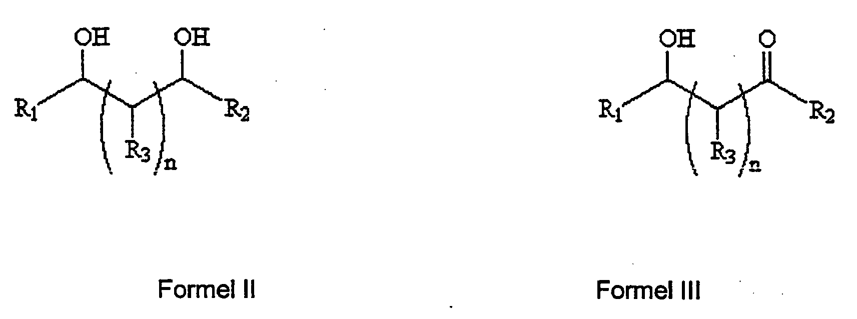

Hierbei haben R₁, R₂, R₃ und n dieselbe Bedeutung wie in Formel I. Überraschend ermöglicht die vorliegende Erfindung hier eine Darstellung verschiedener Stereoisomere in optisch reiner Form.
Je nach Konfiguration an den Stereozentren können die erfindungsgemäß hergestellten Hydroxyverbindungen der Formeln II und III zielgerichtet bei der Synthese chiraler Naturstoffe, Pharma- und Agrowirkstoffe sowie Katalysatoren eingesetzt werden. Beispiele hierfür sind in *Burk*, *M*. *et al*. Tetrahedron Asymmetry (1991) Vol. 2, 569-592, *McKinstry*, *L*. *et al*., Tetrahedron (1995), Vol. 51, 7655-7666, *Julienne K., et al*., J. Org. Chem. (1998), vol. 63, 4532-4534 und *Short*, *R.P. et al*., J. Org. Chem. (1989), Vol. 54, 1755-1756 beschrieben. Hierzu zählen u.a. Tris(phospholane)-Liganden, chirale Phosphine, Thiolane, Pyrrolidine sowie Me-DuPHOS.
Die vorliegende Erfindung ermöglicht bei der Reduktion insbesondere die regioselektive Einführung von *R*- und *S*-Konfigurationen der Hydroxylgruppe.
Das erfindungsgemäße Verfahren wird mit Lactobacillus-Arten durchgeführt. In Betracht kommen grundsätzlich alle Lactobacillus-Arten. Besonders bevorzugt ist jedoch die Verwendung von Lactobacillus kefir und Lactobacillus brevis. Außerdem kann das Fermentationsmedium weitere Mikroorganismen oder Enzyme enthalten.

Überraschend hat sich gezeigt, daß die Stämme der Gattung Lactobacillus in der Lage sind, Verbindungen, die wenigstens zwei Ketogruppen enthalten, zu reduzieren. Beispielsweise kann Hexandion durch Umsetzung mit Lactobacillus-Arten mit sehr hoher Enantiomerenreinheit zu (*R*,*R*)-Hexandiol reduziert werden. Diese Umsetzungen werden zudem mit hoher Reaktionsgeschwindigkeit durchgeführt. Zieht man zum Vergleich die zahlreichen publizierten Reduktionen von Keton-Substraten mit Bäckerhefe heran, ist zu erkennen, daß dort Ketone häufig in Konzentrationen von ca. 1-10g/l eingesetzt werden und daß für diese Umsetzungen mehrere Tage benötigt werden. Hinzu kommt, daß diese Reaktionen noch unvollständig sind. Lactobacillus kefir ist dagegen z.B. in der Lage, Hexandion in einer Konzentration von 5,9 g/l (= 50mM) innerhalb von 3,5 h vollständig umzusetzen.
Das erfindungsgemäße Verfahren kann unter üblichen Fermentationsbedingungen und in den üblichen Reaktoren durchgeführt werden.
Als Cosubstrat kann ein leicht metabolisierbares Kohlenstoff-haltiges Substrat, beispielsweise Glucose verwendet werden. Dadurch werden die bei der Umsetzung verbrauchten Reduktionsäquivalente nachgeliefert.
Die Reaktionen werden erfindungsgemäß bei Temperaturen von 10 bis 50 °C, bevorzugt von 15 bis 40 °C eingestellt.
Der pH-Wert liegt bei 2 bis 10, bevorzugt zwischen 4 und 8. Zur Sicherstellung eines geeigneten pH-Wertes kann jede in der Fermentationstechnik oder Enzymtechnik übliche Puffersubstanz eingesetzt werden. Dies sind z. B. Triethanolamin, Phosphatpuffer, Phosphat-CitratPuffer, 2-Amino-2-(hydroxymethyl)-1,3-propandiol-Puffer, 2-[N-Morpholino]ethansulfonsäure-Puffer (MES) oder Tris-Puffer. Die Konzentrationsbereiche für die Puffer liegen vorteilhafter Weise zwischen 50 und 500 mmol/l.

Erfindungsgemäß hat sich zudem überraschend gezeigt, daß es unter bestimmten Reaktionsbedingungen möglich ist, die Reduktion von Diketonen auf der Stufe des mono-reduzierten Zwischenprodukts anzuhalten, so daß damit enantiomerenreine Hydroxy-Ketone gewonnen werden können. Dies ist insbesondere der Fall, wenn der pH-Wert > 7 ist. Höchst bevorzugt sind daher pH-Werte von 7 bis 8.
Als Reaktoren können ebenfalls alle bekannten Reaktortypen Verwendung finden. So können sowohl herkömmliche Rührreaktoren als auch Festbettreaktoren, in diskontinuierlicher oder kontinuierlicher Betriebsweise sowie in beliebigen Kombinationen dieser Reaktortypen eingesetzt werden.
Bei Anwendung des erfindungsgemäßen Verfahrens entfallen vor allem kostenintensive und umweltbelastende Racemat- oder Diastereomerentrennungen. Die in einer diastereoselektiven Synthese erforderliche Anbindung und Abspaltung einer stöchiometrischen Menge einer homochiralen Hilfsgruppe wird vermieden. Weiterhin ist das Kohlenstoffgerüst der Polyhydroxyverbindungen nach Formel II in den Ausgangsverbindungen nach Formel I bereits komplett, das heißt diese Stereozentren werden erst zu einem späteren Zeitpunkt in die Gesamtsynthesesequenz eingeführt. Dadurch wird der Verlust an homochiralem Material niedrig gehalten.

Im folgenden wird die Erfindung anhand von Beispielen näher beschrieben:

### Beispiel 1: Herstellung von Lactobacillus-Zellen (Lactobacillus kefir)

Zellen für die Reduktion können erhalten werden, indem eine Stammkultur von Lactobacillus kefir (z.B. DSM 20587) in folgendem Medium vermehrt wird:
Pro 1 L: 10 g Caseinpepton, tryptisch verdaut; 10 g Fleischextrakt; 5 g Hefeextrakt, 20 g Glucose; 1 g Tween 80; 2 g K₂HPO₄; 5 g Na-acetat; 2 g Diammoniumcitrat; 0,2 g MgSO₄ x 7 H₂O; 0,05 g MnSO₄ x H₂O; pH = 6,2-6,5. Nach 24 Std. Wachstum werden die Zellen durch Zentrifugation geerntet. Sie können durch Einfrieren gelagert werden.

### Beispiel 2: Herstellung von Lactobacillus brevis-Zellen

Lactobacillus brevis (z.B. DSM 20054) kann in einem Nährmedium wie in Beispiel 1 beschrieben angezogen werden. Nach 48 Std. werden die Zellen durch Zentrifugation geerntet. Sie können durch Einfrieren gelagert werden.

### Beispiel 3: Herstellung von enantiomerenreinem (2R,5R)-Hexandiol

Lactobacillus kefir (DSM 20587) wurde gemäß Beispiel 1 angezogen, mit Kaliumphosphatpuffer (0,1 M; pH 7,0) gewaschen, das Sediment mit diesem Puffer resuspendiert und für die Umsetzung von Hexandion eingesetzt.

| | |
|---|---|
| Ansatz: | 90 ml Zellensuspension |
| | 10 ml Glucose-Lösung (5 molar) |
| | 5 mmol Hexandion |

Nach 60, 90, 120, 180 und 210 min wurde jeweils eine Probe entnommen und mittels gaschromatographischer (GC-) Analyse der Gehalt an restlichem Substrat, an monoreduziertem Zwischenprodukt 2-Hydroxy-2-hexanon und 2,5-Hexandiol ermittelt

### Analysenbedingungen für die Gaschromatographie:

Säule: FS-CW 20M-CB-0.5, 25m x 0,32 mm i.d. (Fa. Macherey und Nagel); Trägergas: Helium; Injektionstemperatur: 250°C; FID: 250°C; Injektionsvolumen: 1µl; Säulentemperatur: 140°C.

Tabelle 1 zeigt die Ergebnisse dieser gaschromatographischen Analytik. Daraus geht hervor, daß das eingesetzte Dion nach 210 min praktisch vollständig zum Hexandiol umgesetzt worden ist.

**Tab 1: Zeitabhängigkeit der Bildung von Hexandiol und dem Zwischenprodukt Hydroxyhexanon bei der Umsetzung mit Zellen von Lactobacillus kefir.**

| Zeit (min) | Hexandion (%) | Hydroxyhexanon(%) | Hexandiol (%) |
|---|---|---|---|
| 0 | 100 | 0 | 0 |
| 60 | 37 | 59 | 4 |
| 90 | 21 | 66 | 13 |
| 120 | 12 | 63 | 24 |
| 180 | 1 | 12 | 87 |
| 210 | 0 | 1 | 99 |

Das nach 210 min Umsetzung vorliegende Produkt wird nach Derivatisierung gaschromatographisch bezüglich der Enantiomerenreinheit überprüft. Dazu wird die Probe mit Chloroform extrahiert, 100 µl Trifluoressigsäureanhydrid zur Chloroform-Phase zugesetzt, 5 min leicht erwärmt und ein Aliquot der Chloroform-Phase für die GC-Analyse eingesetzt.

### Trennbedingungen der Gaschromatographie (Enantiomerentrennung):

Säule: Lipodex E (Fa. Macherey und Nagel, Düren);
Trägergas: Wasserstoff; Säulentemperatur: 90°C.

Unter diesen Bedingungen sind (*R*,*R*)-, (*S*,*S*)- und die meso-Verbindung (*R*,*S*)-Hexandiol trennbar. Das Ergebnis zeigt, daß unter o.a. Bedingungen ausschließlich (2*R*,5*R*)-Hexandiol gebildet wurde, weder (*S*,*S*)- noch (*R*,*S*)-Hexandiol waren nachweisbar.

### Beispiel 4: Optimierung des pH-Werts für die Umsetzung von 2,5-Hexandiol

Zur Bestimmung des optimalen pH-Werts für die Umsetzung von 2,5-Hexandion zu 2,5-Hexandiol bzw. 2-Hydroxy-5-hexanon wurden 3 parallele Ansätze gemäß Beispiel 2 verwendet, lediglich die pH-Werte wurden unterschiedlich eingestellt auf pH 6,0; 7,0 und 8,0. Die pH-Werte wurden alle 30 min gemessen, wobei leichte Abweichungen auf Grund einer Säuerung während der Reaktion (pH 7,0- und 8,0- Ansatz) durch Zusatz von KOH korrigiert wurden. Proben nach 120 min wurden mittels GC entsprechend Beispiel 2 analysiert, die Werte für das Hexandion, 2-Hydroxy-5-hexanon und 2,5-Hexandiol sind in Tabelle 2 zusammengestellt.

**Tab. 2: Gehalt an Hexandion, Hydroxyketon und Hexandiol in Proben aus Umsetzungen bei verschiedenen pH-Werten (120 min-Proben; GC-Analytik)**

| pH-Wert | Hexandion | Hydroxyhexanon | Hexandiol |
|---|---|---|---|
| 6,0 | 1 % | 0% | 99% |
| 7,0 | 4% | 34% | 62% |
| 8,0 | 76% | 24% | <1% |

Die Tabelle 2 zeigt, daß die Reaktion stark pH-abhängig ist, die höchste Ausbeute an Hexandiol (99%) erhält man, wenn die Reaktion bei pH 6,0 abläuft, nahezu kein Diol wird dagegen bei pH 8,0 gebildet.

Dieses Beispiel zeigt zudem eine Möglichkeit zur Herstellung von 2-Hydroxy-5-hexanon auf: Wird die Reaktion bei pH 8,0 durchgeführt, bildet sich zwar das monoreduzierte Hydroxyhexanon, die Weiterreaktion zum Diol läuft allerdings nicht mehr ab, so daß unter diesen Bedingungen 2-Hydroxy-5-hexanon aus Hexandion hergestellt werden kann. Da man als Diol-Produkt enantiomerenreines (2*R*,5*R*)-Hexandiol und keine meso-Verbindung (2*S*, 5*R*)-Hexandiol erhält, muß auch das monoreduzierte Zwischenprodukt enantiomerenrein sein. Man erhält also unter Reduktionsbedingungen bei pH 8,0 (2*R*)-Hydroxy-5-hexanon.

### Beispiel 5: Herstellung von (3R,6R)-Octandiol

Zellen von Lactobacillus kefir DSM 20587 (s. Beispiel 1) wurden in folgendem Ansatz für die Umsetzung von (3,6)-Octandion eingesetzt:

| | |
|---|---|
| Ansatz: | 2 ml Zellensuspension (0,8 g Zellen (Feuchtgewicht) in 0,5 M Kaliumphosphatpuffer, pH 6,0; 100mM Glucose) 10 mM (3,6)-Octandion |

Nach 120 min Inkubation wurde ein Probe entnommen, die Bestimmungen mittels GC-Analytik ergab eine Umsetzung von 69%. Die GC-Analytik zur Bestimmung der Enantiomerenreinheit zeigte, daß als Produkt zu 98,8% (3*R*,6*R*)-Octandiol und zu 1,2% das meso-Produkt gebildet worden ist.

## Patentansprüche

1. Verfahren zur Reduktion von wenigstens zwei Ketogruppen enthaltenden und carbonsäuregruppen- oder estergruppenfreien Verbindungen, bei dem in Gegenwart von Lactobacillus-Arten wenigstens eine Ketogruppe zu einer Hydroxylgruppe umgesetzt wird.

2. Verfahren nach Anspruch 1 **dadurch gekennzeichnet, daß** estergruppenfreie Verbindungen eingesetzt werden.

3. Verfahren nach einem der Ansprüche 1 oder 2, daß wenigstens zwei Ketogruppen zu Hydroxylgruppen umgesetzt werden.

4. Verfahren nach einem der Ansprüche 1 bis 3 **dadurch gekennzeichnet, daß** zu Diolen reduziert wird.

5. Verfahren nach Anspruch 4 **dadurch gekennzeichnet, daß** Verbindungen der Formel I 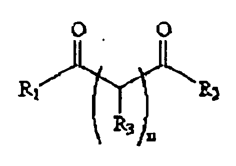 umgesetzt werden, wobei R₁,R₂ = eine Komponente aus der Gruppe Alkyl, Alkenyl, Cycloalkyl, Cycloalkenyl, Aryl, Aralkyl, Cycloalkylalkyl, wobei die Komponenten auch mit Heteroatomen, wie z.B. Si, N, P, O, S, F, Cl, Br oder I, substituiert sein können.
R₃ = R₁, R₂, H, Halogene oder andere Heterokomponenten, z.B. Amine
n = 0 - 10

6. Verfahren nach einem der Ansprüche 1 bis 5 **dadurch gekennzeichnet, daß** Verbindungen der Formel I zu Verbindungen der Formel II, III 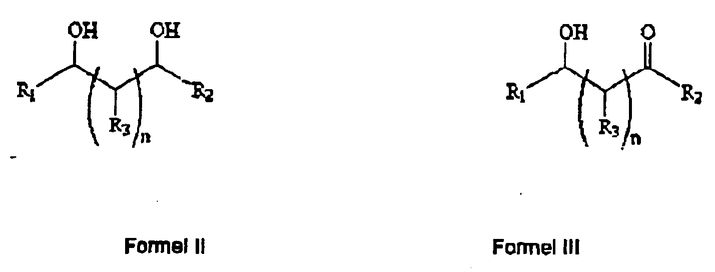 umgesetzt werden, wobei R₁, R₂, R₃ und n dieselbe Bedeutung wie in Formel I haben.

7. Verfahren nach einem der Ansprüche 1 bis 6 **dadurch gekennzeichnet, daß** es bei einem pH-Wert von 2 bis 10 durchgeführt wird.

8. Verfahren nach einem der Ansprache 1 bis 7 **dadurch gekennzeichnet, daß** es bei einem pH-Wert von 4 bis 8 durchgeführt wird.

9. Verfahren nach einem der Ansprüche 1 bis 8 **dadurch gekennzeichnet, daß** Lactobacillus kefir oder Lactobacillus brevis oder eine Mischkultur dieser Mikroorganismen eingesetzt wird.

10. Verfahren nach einem der Ansprüche 1 bis 9 **dadurch gekennzeichnet, daß** zusätzlich zu den Lactobillus-Arten weitere Mikroorganismen oder Enzyme eingesetzt werden.

## Claims

1. Process for reducing compounds which contain at least two keto groups and are free from carboxylic acid groups or ester groups, in which, in the presence of Lactobacillus species, at least one keto group is converted to a hydroxyl group.

2. Process according to Claim 1, **characterized in that** ester-group-free compounds are used.

3. Process according to one of Claims 1 or 2, **characterized in that** at least two keto groups are converted to hydroxyl groups.

4. Process according to one of Claims 1 to 3, **characterized in that** reduction to diols takes place.

5. Process according to Claim 4, **characterized in that** compounds of the formula I 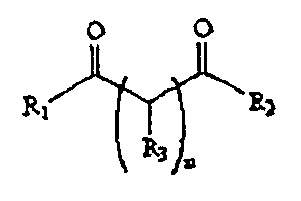 are reacted, where R₁,R₂ = a component from the group alkyl, alkenyl, cycloalkyl, cycloalkenyl, aryl, aralkyl, cycloalkylalkyl, where the components may also be substituted by heteroatoms, such as, for example, Si, N, P, O, S, F, Cl, Br or I,
R₃ = R₁, R₂, H, halogens or other hetero components, e.g. amines
n = 0 - 10.

6. Process according to one of Claims 1 to 5, **characterized in that** compounds of the formula I are converted to compounds of the formula II, III 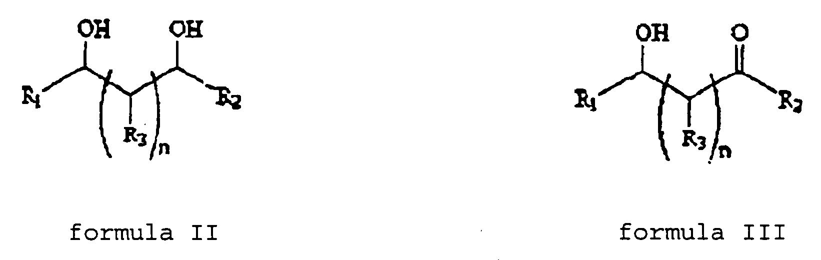 where R₁, R₂, R₃ and n have the same meaning as in formula I.

7. Process according to one of Claims 1 to 6, **characterized in that** it is carried out at a pH of from 2 to 10.

8. Process according to one of Claims 1 to 7, **characterized in that** it is carried out at a pH of from 4 to 8.

9. Process according to one of Claims 1 to 8, **characterized in that** Lactobacillus kefir or Lactobacillus brevis or a mixed culture of these microorganisms is used.

10. Process according to one of Claims 1 to 9, **characterized in that**, in addition to the Lactobacillus species, further microorganisms or enzymes are used.

## Revendications

1. Procédé pour la réduction de composés contenant au moins deux groupes céto et exempts de groupes carboxy ou de groupes ester, dans lequel au moins un groupe céto est converti, en présence d'espèces de *Lactobacillus,* en un groupe hydroxy.

2. Procédé selon la revendication 1, **caractérisé en ce qu'**on utilise des composés exempts de groupes ester.

3. Procédé selon la revendication 1 ou 2, **caractérisé en ce qu'**au moins deux groupes céto sont convertis en groupes hydroxy.

4. Procédé selon l'une quelconque des revendications 1 à 3, **caractérisé en ce qu'**on effectue une réduction en diols.

5. Procédé selon la revendication 4, **caractérisé en ce qu'**on fait réagir des composés de formule I 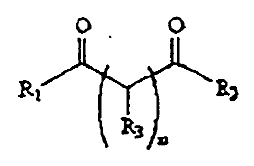 dans laquelle R₁, R₂ représentent un composant choisi parmi des groupes alkyle, alcényle, cycloalkyle, cycloalcényle, aryle, aralkyle, cycloalkylalkyle, les composants pouvant également être substitués par des hétéroatomes, comme par exemple Si, N, P, O, S, F, Cl, Br ou I,
R₃ représente R₁, R₂, des atomes d'hydrogène ou d'halogène ou d'autres hétérocomposants, par exemple des amines,
n va de 0 à 10.

6. Procédé selon l'une quelconque des revendications 1 à 5, **caractérisé en ce que** des composés de formule I sont convertis en composés de formule II ou III 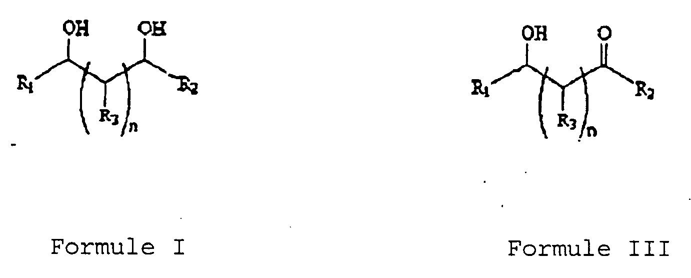 R₁, R₂, R₃ et n ayant la même signification que dans la formule I.

7. Procédé selon l'une quelconque des revendications 1 à 6, **caractérisé en ce qu'**on l'effectue à un pH de 2 à 10.

8. Procédé selon l'une quelconque des revendications 1 à 7, **caractérisé en ce qu'**on l'effectue à un pH de 4 à 8.

9. Procédé selon l'une quelconque des revendications 1 à 8, **caractérisé en ce qu'**on utilise *Lactobacillus* kefir ou *Lactobacillus brevis* ou une culture mixte de ces micro-organismes.

10. Procédé selon l'une quelconque des revendications 1 à 9, **caractérisé en ce qu'**en plus des espèces de Lactobacillus on utilise d'autres micro-organismes ou des enzymes.
